# EUROPEAN PATENT APPLICATION

(11) **EP 4 292 706 A1**
(43) Date of publication of application: **20.12.2023**
(21) Application number: 22752017.8
(22) Date of filing: 13.01.2022
(51) Int. Cl.: B01J 31/02, C07C 6/00, C07C 11/02, C10G 3/00, C07C 51/00, C07C 69/00, C07C 67/00, C07C 31/04, C07D 247/02, C07D 233/02, C07D 307/82, B01J 21/00, B01J 23/44

(54) **METHOD FOR THE COPRODUCTION OF C10 TO C13 OLEFINS AND ESTERS FROM FATTY ACID METHYL ESTERS**

(30) Priority: 11.02.2021 BR 102021002671
(71) Applicant: Petroleo Brasileiro S.A. - PETROBRAS, 20031912 Rio de Janeiro (BR); Universidade Federal De Minas Gerais - UFMG, 31270-901 Belo Horizonte - MG (BR)
(72) Inventor: RENE KLOTZ RABELLO, Carlos, 21941-915 Rio de Janeiro (BR); NICOLAU DOS SANTOS, Eduardo, 31270-901 Belo Horizonte (BR); ALVES FERREIRA, Leonildo, 31270-901 Belo Horizonte (BR); BATISTA DE OLIVEIRA, Kelley Cristina, 31270-901 Belo Horizonte (BR); REBUITI PASSOS, Lucas Henrique, 31270-901 Belo Horizonte (BR); GOMES JUNIOR, Marlito, 21941-915 Rio de Janeiro (BR); VICARI GRANATO, Artur, 31270-901 Belo Horizonte (BR)
(74) Representative: Clarke, Modet y Cía., S.L.
(86) International application number: PCT/BR2022/050012
(87) International publication number: WO 2022/170408

(57) **Abstract**

The present invention addresses to a process for producing olefins and esters in the C10 to C13 range from fatty acid esters through a catalytic hydrogenation reaction followed by cross-metathesis of the hydrogenated product with light olefins.

## Description

### Field of the Invention

The present invention addresses to a process for the production of olefins and esters of the C10 to C13 range from fatty acid esters through a catalytic hydrogenation reaction followed by the cross-metathesis reaction of the hydrogenated product with light olefins of the range from C2 to C6.

### Description of the State of the Art

The metathesis of vegetable oils and their derivatives (FAME - FATTY ACID METHYL ESTERS with C16-C18 chains) with low molecular weight olefins (C2-C4 chains) is an industrial route for obtaining olefins and esters of smaller chains, in the range of C7 to C15. The technology employs metathesis catalysts that are ruthenium complexes in solution. The use of the technology is subject to restrictions regarding the type of inputs. The presence of FAME with di- and tri-unsaturated chains leads to the formation of a wide variety of products and gums. The selective hydrogenation of chains with two and three unsaturations to chains with one unsaturation is an existing solution. However, in this technique, the isomerization of double bonds occurs concomitantly, which greatly increases the number of formed products (loss of selectivity). One of the objectives of this invention is to carry out the treatment by hydrogenation, choosing the catalysts and reaction conditions in such a way that selective hydrogenation occurs without parallel isomerization occurring or that it is minimized. The used metathesis catalysts (complexes of ruthenium with alkylidene ligand) are sensitive to impurities such as peroxides and water. One of the objectives of this invention is also to develop low-cost treatments for the abatement of relevant impurities in FAME, mainly derived from soy oil and palm oil.

In certain industrial processes, the cross-metathesis of vegetable oils with short-chain olefins (C2-C4) is practiced in order to obtain olefins and esters of C10-C13 chains. These processes can be generically called alkenolysis, and the most relevant are those that employ ethene (ethenolysis) and butene (butenolysis). Alkenolysis is usually performed before transesterification, directly on the oil (triglycerides). However, performing it after transesterification (in FAME) may have technical advantages over the direct metathesis of vegetable oils, such as: lower viscosity of the load, less gum formation, easier treatment of the load.

The presence of at least one carbon-carbon double bond in the chain is required for alkenolysis to occur. However, both in the direct metathesis of vegetable oils and in the metathesis of their FAMEs, the presence of carbon chains with more than one carbon-carbon double bond is harmful to the process, as it greatly increases the number of formed derivatives, decreasing the yield of certain products and making it difficult to purify the products. The presence of di-(C18:2) and tri-(C18:3) unsaturated chains is common in oils such as soybean oil. A process in which these di-(C18:2) and tri-(C18:3) unsaturated chains are selectively hydrogenated to monounsaturated (C18:1) chains prior to the metathesis process is desirable. However, the hydrogenation catalyst and the reaction conditions must be rigorously chosen, in order to avoid total hydrogenation (formation of saturated C18:0 chains) as well as position isomerization in the double bonds, which leads to a great increase in products and, consequently, in the loss of selectivity and difficulty in purifying the products. In the metathesis step, the used catalyst is a ruthenium alkylidene complex that is deactivated by impurities in the load such as peroxides, oxygen and water. These impurities must be removed, but the technique used to remove them must be of low-cost, under penalty of making the process economically unfeasible. The average number of catalytic cycles for the process to be viable must exceed 40,000.

Thus, the technology presented here is useful for the pretreatment of FAMEs rich in polyunsaturated chains such as soybean FAME. Even FAMEs with lower levels of polyunsaturated chains can benefit from this treatment, since the formation of derivatives from the small amounts of di- and tri-unsaturated chains make the purification of the main products very difficult. In addition, the hydrogen treatment itself helps to reduce relevant impurities such as peroxides and waste water.

The alkenolysis of vegetable oils is a process of high industrial interest and has been the subject of a large number of intellectual property claims.

The alkenolysis of vegetable oils using ruthenium catalysts is described, for example, in WO 2003093215, but is not the object of the present invention. The document cites the use of raw material free of impurities, but without mentioning how to remove the poisons that affect the performance of the metathesis catalyst. According to this document, the fatty acids or esters must have less than 1 to 3 meq per kilogram of catalyst referring to poisons such as organic hydroperoxides.

The alkenolysis of FAMEs derived from vegetable oils using ruthenium catalysts is described, for example, in documents US 7,119,216; US 8,501,973; US 2013/0035502 and in the paper by NICKEL, A; UNG, T; MKRTUMYAN, G; et al "A Highly Efficient Olefin Metathesis Process for the Synthesis of Terminal Alkenes from Fatty Acid Esters", Top. Catal. 55:518-523 (2012), but are not objects of the present invention.

Document US 7,119,216 uses osmium or ruthenium catalysts supported on a polymeric resin. Document US 8,501,973 teaches the metathesis of vegetable oil or ester with ethylene, with a previous treatment to eliminate catalyst poisons present in the raw materials. The treatment consists of removing peroxides with different reagents, such as: hydrated magnesium silicate, sodium bisulfite and celite. Document US2013/0035502 discloses the use of two metathesis catalysts for reactions with natural oil, with the second catalyst being based on rhenium or tungsten.

The use of a wide variety of ruthenium complexes containing an alkylidene ligand as effective catalysts for the metathesis of olefins is claimed, for example, in WO 2010/010290 and WO 2011/008258, but the use of these catalysts is not the object of the present invention. It is obvious to experts that a wide variety of ruthenium complexes containing an ylidene-type ligand and at least one electron-density strongly-donor ligand can be used in the alkenolysis of vegetable oils or their derivatives.

The document PI 0208639-5 teaches about a metathesis process of olefins from oleic acid or its methyl ester with catalysts based on ruthenium or osmium supported on various materials (silica, alumina, zirconia and others) with ligands organic, and which can be supported on an ion exchange resin.

Document US 8,614,344 discloses methods for cross-metathesis of compositions of polyunsaturated fatty acids with light olefins, where there is a first step of catalytic hydrogenation aiming at raising the increase in the content of fatty acids and monounsaturated derivatives. However, in the hydrogenation step, migration of the remaining double bonds occurs simultaneously. The catalysts used were preferably ruthenium-based catalysts. Other catalysts mentioned were molybdenum, tungsten, chromium, rhenium and osmium. The catalysts also have organic ligands.

The Master's Thesis (DE SOUZA LIMA, P. Metathesis of olefins with vegetable oils: a strategy for the development of biorefineries. Master's dissertation. UFRS. 75 p. October 2011) studied cross-metathesis reactions of vegetable oils and fatty acid esters obtained by transesterification and esterification with ethylene and Grubbs/Ruthenium catalysts. In addition to products of petrochemical interest such as terminal olefins obtained in the cross-metathesis reaction, reactions for the production of biofuels (biogasoline and biokerosene) were also studied. Biofuels were obtained through hydrogenation and transesterification of terminal olefins.

Document BR 11 2015 021070-8 discloses a method to produce and refine fatty acids and fatty acid esters from oil raw materials through the cross-metathesis of esters derived from fatty acids with low molecular weight olefins, which initially consists of isomerizing the esters and then conducting the metathesis reaction. After that, there is a final step of separating the olefins and also of transesterification of the esters. The metathesis catalyst is not disclosed in BR 11 2015 021070-8, only the isomerization catalyst. The isomerization can be carried out by heat or by means of a ruthenium catalyst, with the formula (PCy₃)₂(Cl)(H)Ru(CO), or a solid acid catalyst, the sulfonated tetrafluoroethylene. Document BR 11 2015 021070-8 does not disclose the removal of impurities such as hydroperoxides or water for the metathesis reaction, nor the low formation of ester isomerization as in the present invention.

The refining of metathesis products from vegetable oils and their derivatives by treatment with hydrogen after the metathesis step is described, for example, in document US 2015/0247107, but is not the object of the present invention. Document US 2015/0247107 teaches the importance of various types of pre-treatment to remove organic peroxides, which are poisons for the metathesis catalyst, such as: zeolites, reaction with metal hydrides, and others.

The selective hydrogenation of di- and tri-unsaturated FAMEs to obtain mono-unsaturated FAMEs with the purpose of improving the load for the alkenolysis process catalyzed by ruthenium complexes has previously been described in documents WO 2008/048522 and US 60/851628. However, in the hydrogenation step, migration of the remaining double bonds occurs simultaneously.

A review of the literature on metathesis via heterogeneous catalysis of esters of unsaturated fatty acids, especially the cross-metathesis of methyl oleate with low molecular weight olefins and the auto-metathesis of methyl oleate was recently published (NIERES, P. D. ; ZELIN, J.; TRASARTI, A. F.; APESTEGUIA, C. R. Valorization of vegetable oils by heterogeneous catalysis via metathesis reactions. Current Opinion in Green and Sustainable Chemistry 10: 1-5, 2018). One of the conclusions of the review is that further studies are still needed to develop stable and regenerable heterogeneous metathesis catalysts for the conversion of unsaturated fatty acid esters to chemical products with higher added value.

It is worth to highlight that, in the hydrogenation processes already disclosed in the state of the art, the remaining double bonds undergo migration in the chain, which generates a larger series of products of lesser interest, reducing the yield for the products and making their separation difficult. In none of the previous processes was selective hydrogenation described through the appropriate choice of catalysts and process conditions, so that the migration of the double bonds in the chain (isomerization) does not occur and that, in addition, this treatment dispenses with additional treatments for the removal impurities that deactivate the metathesis catalyst, such as organic peroxides.

### Brief Description of the Invention

The present invention addresses to a process for the production of olefins and esters, preferably in the range of C10 to C13 from fatty acid esters through a catalytic hydrogenation reaction followed by the cross-metathesis reaction of the hydrogenated product with olefins light from the range of C2 to C6. Shorter chain olefins and longer chain esters can be formed depending on the light olefin used.

The pretreatment of the load by catalytic hydrogenation under conditions such that the hydrogenation of compounds with 2 or 3 C-C double bonds is highly selective, resulting in compounds with a C-C double bond without the isomerization of the double bonds occurring, or that is minimized, has not been described in the state of the art. So that isomerization does not occur, in this invention, catalysts were chosen, as well as the reaction conditions such as temperature, pressure and amount of hydrogen. The treatment with hydrogen is sufficient for the alkenolysis of the FAMEs to occur with high efficiency in the metathesis catalyst. In addition, the process developed herein removes the organic peroxides in the hydrogenation step, which are poisons for the metathesis catalysts and are contaminants usually present in fatty acid esters and vegetable oils.

### Brief Description of the Drawings

The present invention will be described in more detail below, with reference to the attached figures which, in a schematic way and not limiting the inventive scope, represent examples of the same. In the drawings, there are:

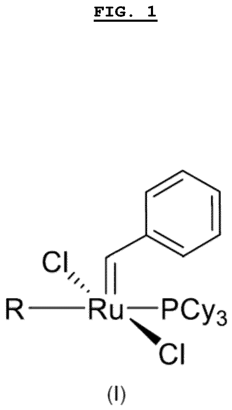

- Figure 1 shows the ruthenium (I)-based metathesis catalyst with the R ligand.

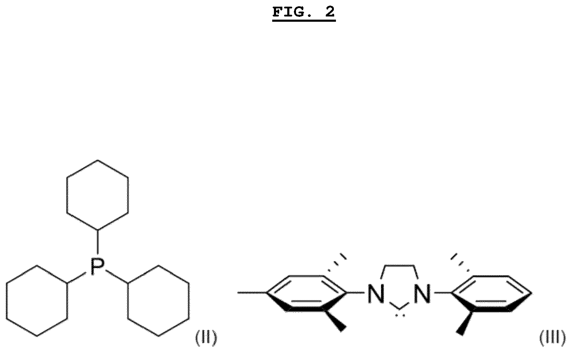

- Figure 2 shows the R ligand referring to the metathesis catalyst of Figure 1, where R can be the chemical structure (II), which corresponds to the structure of the first-generation Grubbs catalysts (GI), or the chemical structure (III), which corresponds to the structure of second-generation Grubbs catalysts (GII).

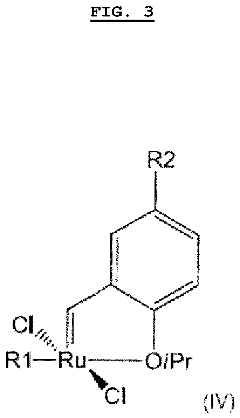

- Figure 3 shows another catalyst for metathesis of olefins based on ruthenium (IV), where R1 and R2 are organic radicals. The radical R2 can be a hydrogen atom (H) or the organic radical NHCO₂*ⁱ*Bu (isobutyl formate).

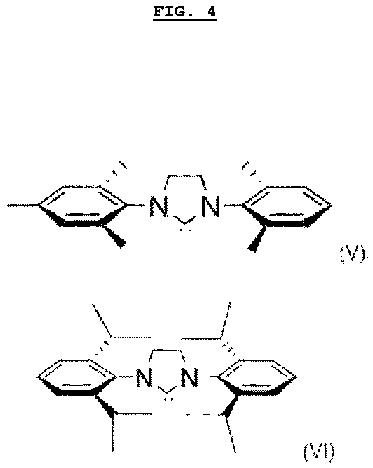

- Figure 4 shows the organic radical R1 of the metathesis catalyst shown in Figure 3. The radical R1 can assume two different chemical structures shown: V and VI. When the radical R1 corresponds to structure V, the catalyst is known as a second-generation Hoveyda-Grubbs catalyst (HGII), and when the radical R1 corresponds to structures VI and VII, the catalysts are known as second-generation Hoveyda-Grubbs analogue catalysts.

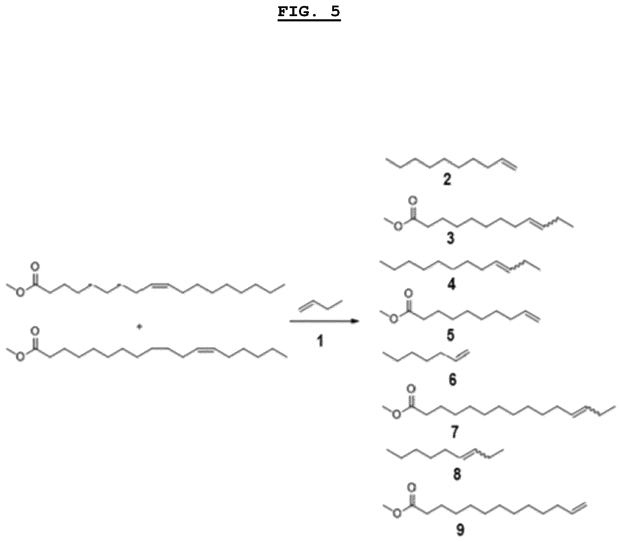

- Figure 5 shows the metathesis reaction with 1-butene (butenolysis) with FAMEs from hydrogen-treated soybean oil (BSPH - Partially Hydrogenated Soybean Biodiesel) in the presence of the metathesis catalyst (1). Figure 5 also shows the expected products without parallel isomerization occurring: 1-decene (2), methyl 12-dodecenoate (3), 3-dodecene (4), methyl 9-decenoate (5), 1-heptene (6), methyl 12-pentadecenoate (7), 3-nonene (8), and methyl 12-tridecenoate (9).

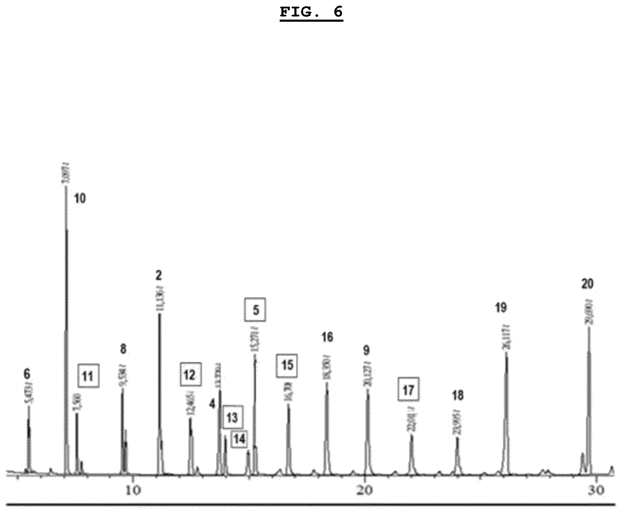

- Figure 6 shows the chromatographic profile obtained by the analysis by gas chromatography of the products obtained in the butenolysis reaction of FAME from hydrogenated soybean oil under conditions in which a significant isomerization occurs, and the products from the isomerization are highlighted with an outline. The peaks present in the chromatogram shown in Figure 6 refer to the following chemical compounds: 1-heptene (6), toluene (10), octene (11), 3-nonene (8), 1-decene (2), 1-undecene (12), 3-dodecene (4), methyl nonenoate (13), tridecene (14), methyl 9-decenoate (5), methyl undecenoate (15), methyl dodecenoate (16), methyl tridecenoate (9), methyl tetradecenoate (17), methyl pentadecenoate (18), methyl palmitate (19), methyl stearate (20).

### Detailed Description of the Invention

Preliminarily, it is emphasized that the description that follows will start from preferred embodiments of the invention. As will be evident to any technician skilled on the subject, however, the invention is not limited to these particular embodiments, but only to the scope of protection defined in the claims.

This work enabled the development of a previous treatment of the load by hydrogenation under conditions, such that the hydrogenation of compounds with 2 or 3 C-C double bonds is highly selective, resulting in compounds with a C-C double bond without the isomerization of the double bonds C-C occurs significantly. To reduce isomerization, in this invention, catalysts were chosen, as well as the reaction conditions such as temperature, pressure and amount of hydrogen. In addition, the treatment with hydrogen is sufficient for the alkenolysis of FAMEs to occur with high efficiency of the metathesis catalyst, without the need for additional treatments to abate relevant impurities.

The process of lysis (breaking) of carbon chains of fatty acid methyl esters (FAME) is carried out through metathesis with olefins of chains in the range of C2 to C6 (alkenolysis), where there is a previous treatment by catalytic hydrogenation of the methyl fatty acid esters. The hydrogenation process takes place selectively with di- and tri-unsaturated chains to produce a mixture of hydrogenated products with monounsaturated chains, and in which the migration from the original position of the double bonds in the carbon chain is less than 5%, which is obtained through the choice of catalysts and process conditions. The FAMEs used in this hydrogenation process followed by metathesis with olefins in the C2 to C6 range are preferably obtained from vegetable oils via transesterification with methanol. It will be apparent to experts that the process could be applied to fatty acid ethyl esters, or even fatty acid triglycerides.

The metathesis catalyst consists of ruthenium, whose chemical structures and radicals are shown in Figures 1 to 4. The carbon chain olefins in the range from C2 to C6 are: ethene, 1-propene, 2-propene, 1-butene, 2-butene, 1-pentene, 2-pentene, 1-hexene, 2-hexene, 3-hexene or mixtures thereof, it being preferable to use 1-butene because it is easier to access and used exclusively for products in the range of interest when reacting with methyl oleate. The olefin content is less important than the existence of relevant impurities. Impurities such as: peroxides, sulfonated elements, and others can be harmful even at levels below 100 ppm (0.01%). The short chain olefin must have impurity contents less than 100 ppm, preferably less than 10 ppm.

The process is conducted in four main steps:
a) catalytic hydrogenation of methyl fatty acid esters with di- and tri-unsaturated chains;
b) the lysis (or breaking) of the carbonic chains of methyl esters of the hydrogenated mixture by the cross-metathesis reaction with olefins of carbonic chains in the range of C3 to C6;
c) separation of esters and olefins from the C10 to C13 chain by fractional distillation.

The hydrogenation step is conducted in a batch reactor or, even, mixing reactor (CSTR - Continuous Stirred Tank Reactor), at pressures from 5 to 80 bar (0.5 to 8 MPa), with a temperature in the range of 30 to 80 °C, with stirring of 10 to 2,000 rpm (revolutions per minute), space velocities from 0.1 to 10 h⁻¹ or stirring time between 0.01 and 24 h. This step is preferably carried out in a mixture-type reactor (CSTR), at a temperature of 50 °C, with 500 rpm of stirring and 0.5 h of stirring time. Alternatively, a fixed bed reactor can be used in the same ranges of the mentioned variables.

Decreasing temperature tends to reduce isomerization. It will be evident to specialists that temperatures even lower than those reported can be used with benefits for selectivity, but at the expense of reaction speed. Hydrogen pressure appears to have little influence on isomerization for certain catalysts. Pressures from 15 bar (1.5 MPa) to 80 bar (8 MPa) can be used, but the reaction must be stopped when the gauge pressure indicates the consumption of the stoichiometric amount of hydrogen necessary to hydrogenate di- and tri-unsaturated FAMEs to monounsaturated FAMEs. In the experimental arrangement used, this amount is approximately 15 bar (1.5 MPa). It will be obvious to experts that lower hydrogen pressures could be used, provided the reactor is fed back with enough hydrogen to reach the stoichiometric value. Higher hydrogen pressures could also be used as long as there are safety conditions for doing so.

The metathesis reaction of olefins is conducted in a reaction vessel under the following conditions: pressure between 0.1 and 50 bar (0.01 and 5 MPa), stirring of the reaction medium at 10 to 2000 rpm, temperature between -5 and 120 °C, stirring time between 0.01 h and 24 h. This reaction is preferably carried out under the following conditions: pressure at 5 bar (0.5 MPa), stirring of the reaction medium at 500 rpm, temperature at 50 °C, stirring time 0.5 h.

It is known that the first-generation Grubbs catalysts (GI - structure I, ligand II) have a much lower performance than the second-generation Grubbs (GII - structure I - ligand III) in the butenolysis of FAMEs and triglycerides. It is also known that the second-generation Hoveyda-Grubbs catalyst (HGII - structure IV - ligand V) and its analogues (structure IV - ligand VI) have similar activity to the GII catalyst. Furthermore, it is known that several catalysts with structures similar to I and IV are active for this reaction. The metathesis catalysts used were selected among dozens of possibilities, just to demonstrate that with the hydrogenation treatment of FAMEs under the selected conditions, isomerization products of the double bond are not formed, and butenolysis can be performed with a higher number of rotations (TON) to 50 thousand for these catalysts. The TON number specifies the maximum use that can be made of a catalyst, for a given reaction in particular, under the conditions defined by a series of molecular reactions or reaction cycles, which occur in the active center of the catalyst until its activity drops. The use of similar catalysts is already known in the state of the art and would be obvious to specialists.

### EXAMPLES:

Below, so that the invention can be better understood, experiments are presented that illustrate the invention, without, however, being considered limiting.

### EXAMPLE 1: Selective hydrogenation of soybean FAME and ethenolysis

The hydrogenation reactions were carried out in a stainless-steel autoclave. A mass of FAMEs containing 17.1 mmol of methyl linoleate (10.3 g of FAMEs) and a mass of catalyst containing 0.005 mmol of palladium were added to a glass beaker. The glass beaker was placed in a stainless-steel reactor, which was pressurized with hydrogen. Finally, the reactor was placed on a heating plate with magnetic stirring, previously heated to the reaction temperature, and the pressure drop was recorded over time through a pressure transducer coupled to a fieldlogger recorder. The products were analyzed by gas chromatography and gas chromatography coupled to mass spectrometry. To determine whether there was the migration of double bonds, the hydrogenation products were separated and subjected to ethenolysis (metathesis with ethene), using the first-generation Grubbs catalyst (GI), although this is much less active and stable than the GII and HGII. The use of second-generation Grubbs and Hoveyda-Grubbs catalysts leads to the isomerization concomitant with the ethenolysis. As the ethenolysis was used here only in the context of an analytical tool to detect isomerization during hydrogenation, the poor performance of the catalyst was not an impediment. Under the tested conditions of ethenolysis, it was determined that the isomerization of the double bond does not occur. Thus, if products only explainable by the isomerization of the double bond occur, this isomerization occurred in the hydrogenation step. The results are shown in Table 1. The low presence of C9, C11, C12 and C14 products, for example, in tests 7 and 8, indicates that an isomerization did not occur in the hydrogenation step. Corroborating the results of the analysis by ethenolysis, the analysis by gas chromatography was performed using a column and chromatographic conditions in which the positional and geometric double bond isomers are separated. This analysis was consistent with the results using the ethenolysis as an analytical tool; that is, the incidence of isomerization products was minimal.

### EXAMPLE 2: butenolysis

Soybean FAME hydrogenated as described in example 1 was subjected to butenolysis, and hydrogenation was the only previous treatment after synthesis of the FAMEs. In a steel reactor containing a magnetic bar coated with PTFE, under an inert atmosphere, 20 mL of the selectively hydrogenated soybean FAME, the second-generation Grubbs catalyst (GII) were introduced in a molar ratio of 15.6 ppm in relation to the number of moles of C-C double bonds. The reactor was closed and cooled to -5 °C (268 K). Five grams of 1-butene (purity greater than 99%) were condensed in the reactor, which was closed and transferred to an aluminum block previously heated to 60 °C under magnetic stirring, where it remained for 60 minutes. The reactor was removed, cooled and excess pressure (if any) was released through a needle valve under exhaustion. An aliquot of the products was taken and analyzed by gas chromatography. The results are presented in Table 2, where the fraction of products containing the ester group is identified.

The metathesis with 1-butene (butenolysis) was performed with hydrogen-treated soybean FAMEs (BSPH). The expected products without parallel isomerization occurring are shown in Figure 5.

Figure 6 shows the chromatographic profile (gas chromatography) of the butenolysis products of hydrogenated soybean FAME under conditions in which a significant isomerization occurs and the products from isomerization are highlighted with an outline. The distribution of unsaturated esters, products of the butenolysis, are shown in Table 2. The presence of methyl undecenoate and methyl tetradecenoate indicates the occurrence of isomerization. Thus, the quantification of these products is a way to monitor the occurrence of the position isomerization and the smaller the sum value, the smaller the occurrence of isomerization. Selected examples of the distribution of products in the olefinic fraction are shown in Table 3.

The pretreatment by hydrogenation under the conditions described in Table 2 in the tests with the Pd-Ag/Al₂O₃ catalyst at 30 °C at 15 and 80 bar (1.5 and 8 MPa) indicate low isomerization, either in the hydrogenation phase or in the butenolysis phase. Since the only treatment is hydrogenation, the GII and HGII catalysts reach rotation numbers (turnover number - TON) greater than 50,000, indicating that the pre-treatment with hydrogen is sufficient to obtain a high performance for the metathesis catalyst.

**Table 1. Chromatographic analysis and ethenolysis of partially hydrogenated soybean FAMEs under different conditions.**

| **Conditions:** ^{a} Soy FAME Hydrogenation = Soy FAME (22.0 mL), 0.005 mmol Pd, 700 rpm. ^{b} Ethenolysis and partially hydrogenated soy FAME (10 mL), G-I (10.0 mg), toluene | | | | | | | | | | | | | | | | | | |
|---|---|---|---|---|---|---|---|---|---|---|---|---|---|---|---|---|---|---|
| **Test** | **Hydrogenation ^{a}** | | | | | | | | | | | **Ethenolysis ^{b}** | | | | | | |
| | **Catalyst** | **P (bar) 1 bar** = **0.1 MPa** | **T (°C)** | **Retention time (min)** | | | | | | | | **Conversion (%)^{c}** | **Distribution of products ⁴ (%)** | | | | | |
| | | | | **18.6 C_{16:0}** | **29.1 C_{18:0}** | **31.5 *t*-C_{18:1}** | **32.6 *c-*C_{18:1}** | **33.2** - | **33.7** - | **34,2** - | **38.6 C_{18:2}** | | **Ca** | **C₁₀** | **C₁₁** | **C₁₂** | **C₁₃** | **C₁₄** |
| FAME (load) | | | | 14 | 6 | 0 | 23 | 0 | 0 | 0 | 51 | --- | | | | | | |
| 1 | Pd/C | 15 | 80 | 13 | 22 | 20 | 31 | 3 | 8 | 0 | 3 | 41 | 2 | 62 | 5 | 6 | 23 | 2 |
| 2 | Pd-Ag/ Al₂D₃ | 15 | 80 | 13 | 11 | 21 | 31 | 2 | 14 | 0 | 0 | 48 | 1 | 55 | 3 | 5 | 34 | 1 |
| 3 | Pd/Al₂O₃ | 15 | 80 | 12 | 16 | 40 | 25 | 3 | 4 | 1 | 0 | 35 | 8 | 32 | 16 | 14 | 23 | 7 |
| 4 | Pd/Al₂O₃ | 15 | 50 | - | - | | - | - | - | - | - | 45 | 2 | 55 | 6 | 8 | 27 | 2 |
| 5 | Pd/Al₂O₃ | 15 | 30 | 12 | 11 | 17 | 42 | 2 | 15 | 0 | 1 | 49 | 1 | 57 | 3 | 5 | 34 | 1 |
| 6 | Pd/Al₂O₃ | 15 | 20 | 12 | 11 | 18 | 42 | 2 | 15 | 0 | 0 | 47 | 1 | 56 | 3 | 4 | 35 | 1 |
| 7 | Pd/Al₂O₃ | 80 | 20 | 13 | 12 | 10 | 41 | 2 | 16 | 0 | 7 | 47 | 0 | 67 | 1 | 3 | 29 | 0 |
| 8 | Pd/Al₂O₃ | 80 | 30 | 13 | 12 | 9 | 45 | 1 | 19 | 2 | 0 | 51 | 0 | 62 | 1 | 3 | 33 | 0 |

(4.5 mL), ethylene (4.0 bar (0.4 MPa)), 50 °C, 4 h, 500 rpm. ^{c} Conversion, calculated by gas chromatography using the area of methyl palmitate as an internal standard. ^{d} Considering only products containing an ester function. ^{e} Initial pressure of 80 bar (8 MPa) and the reaction was stopped after consuming 16 bar (1.6 MPa) of H₂.

**Table 2. Soybean FAME hydrogenation followed by butenolysis.**

| Notes: 1- Products derived from isomerization: I1 + I2. 2- Hydrogenation condition: 22 | | | | | | | | | | | |
|---|---|---|---|---|---|---|---|---|---|---|---|
| N | **Hydrogenation Catalyst** | **Hydrogenation Condition 1 bar m 0,1 MPa** | **Catalyst for butenolysis** | **Butenolysis conversion (%)** | **Distribution of butenolysis esters (%)** | | | | | | ***l1* + *l2*** |
| | | | | | **methyl decenoate** | **methyl undecenoate (*l*1)** | **methyl dodecenoate** | **methyl tridecenoate** | **methyl tetradecenoate (*l*2)** | **methyl pentadecenoale** | |
| 1 | Pd/Al₂O₃ | **A** 80°C 15 bar | **Grubbs II (structure I - ligand II)** | 98 | 20 | 15 | 22 | 22 | 12 | 10 | 27 |
| 2 | Pd-Ag/Al₂O₃ | **B** 80°C 15 bar | | 90 | 29 | 5 | 24 | 22 | 5 | 14 | 10 |
| 3 | Pd/C | **C** 80°C 15 bar | | 98 | 28 | 9 | 24 | 19 | 9 | 12 | 18 |
| 4 | Pd/Al₂O₃ | **D** 30°C 80 bar | | 92 | 26 | 9 | 22 | 22 | 9 | 12 | 18 |
| 5 | Pd/Al₂O₃ | **E** 30°C 80 bar ⁴ | | 99 | 28 | 8 | 22 | 23 | 7 | 12 | 15 |
| 6 | Pd-Ag/Al₂O₃ | **F** 30°C 80 bar | | 92 | 35 | 2 | 25 | 23 | 2 | 14 | 4 |
| 7 | Pd-Ag/AL₂O₃ | **G** 30°C 80 bar⁴ | | 96 | 35 | 2 | 23 | 23 | 2 | 15 | 4 |
| 8 | Pd-Ag/Al₂O₃ | **H** 30°C 15 bar | | 90 | 29 | 2 | 29 | 20 | a | 19 | 4 |
| 9 | Pd/C | **I** 30°C 80 bar | | 81 | 33 | 4 | 25 | 21 | 4 | 14 | 8 |
| 10 | Pd/C | **J** 30°C 80 bar ⁴ | | 84 | 33 | 4 | 25 | 21 | 4 | 14 | 8 |
| 11 | Pd/Al₂O₃ | **K** 30°C 80 bar | **Hoveyda-Grubbs II (structure IV, ligand V)** | 92 | 29 | 5 | 24 | 20 | 7 | 15 | 12 |
| 12 | Pd/C | **L** 30°C 80 bar | | 91 | 33 | 2 | 26 | 21 | 4 | 14 | 6 |
| 13 | Pd-Ag/Al₂O₃ | **M** 30°C 80 bar | | 97 | 36 | 2 | 22 | 24 | 2 | 15 | 4 |

mL of soybean biodiesel; catalyst: mass equivalent to 0.01 mmol of Pd; reaction stopped after consumption of the stoichiometric amount of hydrogen. 3- Butenolysis conditions: 20 ml of partially hydrogenated soybean biodiesel (BSPH), 5 g of butene, 60 °C, 2 h, amount of catalyst: 15.6 ppm (relative to the number of double bonds in the used BSPH). 4- vacuum distilled BSPH before the butenolysis reaction.

**Table 3: Selected examples of the distribution of products in the olefinic fraction for the hydrogenation of soybean FAME followed by butenolysis**

| **Test** | **Distribution of the olefinic fraction (%)** | | | | | |
|---|---|---|---|---|---|---|
| | **heptene** | **octene** | **nonene** | **1-decene** | **undecene** | **dodecene** |
| 4 | 13 | 7 | 15 | 35 | 11 | 20 |
| 6 | 15 | 2 | 13 | 42 | 2 | 27 |
| 9 | 15 | 2 | 13 | 40 | 4 | 26 |

| | | | | | | |
|---|---|---|---|---|---|---|
| Note: 1- reaction conditions: same as in Table 2 present in notes 2 and 3. | | | | | | |

## Claims

1. **A PROCESS FOR THE COPRODUCTION OF OLEFINS AND C10 TO C13 ESTERS FROM METHYL FATTY ACID ESTERS**, **characterized in that** it comprises the following steps:
a) catalytic hydrogenation of methyl fatty acid esters with di- and tri-unsaturated chains;
b) the lysis of the carbonic chains of methyl esters of the hydrogenated mixture by the cross-metathesis reaction with olefins of carbonic chains in the range of C2 to C6;
c) separation of esters and olefins from the C10 to C13 chain by fractional distillation.

2. **THE PROCESS FOR THE COPRODUCTION OF OLEFINS AND C10 TO C13 ESTERS FROM METHYL FATTY ACID ESTERS** according to claim 1, **characterized in that** the methyl fatty acid ester is obtained from vegetable oils.

3. **THE PROCESS FOR THE COPRODUCTION OF OLEFINS AND C10 TO C13 ESTERS FROM METHYL FATTY ACID ESTERS** according to claim 1, **characterized in that** the olefin metathesis catalyst has the following chemical structure:

4. **THE PROCESS** 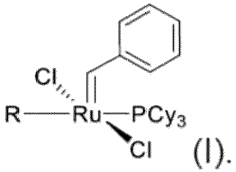 **FOR THE COPRODUCTION OF OLEFINS AND C10 TO C13 ESTERS FROM METHYL FATTY ACID ESTERS** according to claim 3, **characterized in that** the organic ligand R is selected from the following chemical structures:

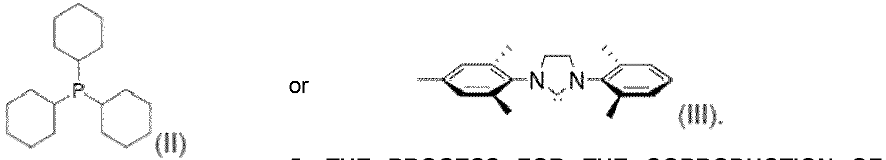

5. **THE PROCESS FOR THE COPRODUCTION OF OLEFINS AND C10 TO C13 ESTERS FROM METHYL FATTY ACID ESTERS** according to claim 1, **characterized in that** the olefin metathesis catalyst has the following structural formula:

6. **THE PROCESS** 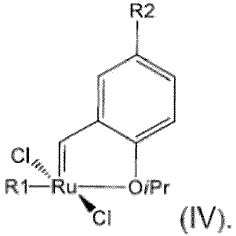 **FOR THE COPRODUCTION OF OLEFINS AND C10 TO C13 ESTERS FROM METHYL FATTY ACID ESTERS** according to claim 5, **characterized in that** the organic ligand R is selected from the following chemical structures:
a. R1 is 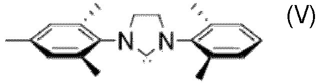 and R2 is hydrogen; or
b. R1 is 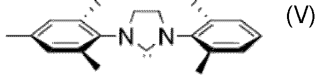 and R2 is NHCO₂*ⁱ*Bu; or
c. R1 is 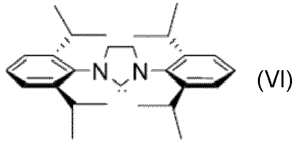 and R2 is NHCO₂*ⁱ*Bu.

7. **THE PROCESS FOR THE COPRODUCTION OF OLEFINS AND C10 TO C13 ESTERS FROM METHYL FATTY ACID ESTERS** according to claim 1, **characterized in that** the olefin is selected from: ethylene, 1-propene, 2-propene, 1-butene, 2-butene, 1-pentene, 2-pentene, 1-hexene, 2-hexene, 3-hexene or mixtures thereof.

8. **THE PROCESS FOR THE COPRODUCTION OF OLEFINS AND C10 TO C13 ESTERS FROM METHYL FATTY ACID ESTERS** according to claim 7, **characterized in that** the selected olefin is preferably 1-butene.

9. **THE PROCESS FOR THE COPRODUCTION OF OLEFINS AND C10 TO C13 ESTERS FROM METHYL FATTY ACID ESTERS** according to claim 1, **characterized in that** the hydrogenation step is conducted in a reactor at pressures from 5 to 80 bar (0.5 to 8 MPa), with temperature in the range of 30 to 80 °C, with stirring from 10 to 2,000 rpm, space velocity from 0.1 to 10 h⁻¹ or stirring time between 0.01 and 24 h.

10. **THE PROCESS FOR THE COPRODUCTION OF OLEFINS AND C10 TO C13 ESTERS FROM METHYL FATTY ACID ESTERS** according to claim 9, **characterized in that** the hydrogenation step is preferably conducted in a reactor at a temperature of 50 °C, with 500 rpm of stirring and 0.5 h of stirring time.

11. **THE PROCESS FOR THE COPRODUCTION OF OLEFINS AND C10 TO C13 ESTERS FROM METHYL FATTY ACID ESTERS** according to claims 1, 9 and 10, **characterized in that** the hydrogenation catalyst is composed of 0.05% m/m to 5.0% m/m metallic palladium and 0.01% m/m to 1% m/m metallic silver deposited on gamma-alumina.

12. **THE PROCESS FOR THE COPRODUCTION OF OLEFINS AND C10 TO C13 ESTERS FROM METHYL FATTY ACID ESTERS** according to claims 1, 9 and 10, **characterized in that** the hydrogenation catalyst is composed of 0.1% m/m to 5% m/m of metallic palladium deposited on active carbon.

13. **THE PROCESS FOR THE COPRODUCTION OF OLEFINS AND C10 TO C13 ESTERS FROM METHYL FATTY ACID ESTERS** according to claims 1, 9 and 10, **characterized in that** the hydrogenation catalyst is composed of a content of 0.05% m/m at 5.0% m/m of metallic palladium deposited on gamma-alumina.

14. **THE PROCESS FOR THE COPRODUCTION OF OLEFINS AND C10 TO C13 ESTERS FROM METHYL FATTY ACID ESTERS** according to claim 11, **characterized in that** the hydrogenation catalyst is preferably composed of 0.1% m/m of doped metallic palladium with 0.01% m/m to 1% m/m of metallic silver deposited on gamma-alumina.

15. **THE PROCESS FOR THE COPRODUCTION OF OLEFINS AND C10 TO C13 ESTERS FROM METHYL FATTY ACID ESTERS** according to claim 12, **characterized in that** the hydrogenation catalyst is preferably composed of 0.5% m/m of metallic palladium deposited on active carbon.

16. **THE PROCESS FOR THE COPRODUCTION OF OLEFINS AND C10 TO C13 ESTERS FROM METHYL FATTY ACID ESTERS** according to claim 13, **characterized in that** the hydrogenation catalyst is preferably composed of a content of 0.7% m/m of metallic palladium deposited on gamma-alumina.

17. **THE PROCESS FOR THE COPRODUCTION OF OLEFINS AND C10 TO C13 ESTERS FROM METHYL FATTY ACID ESTERS** according to claims 1 to 8, **characterized in that** the metathesis reaction of olefins is conducted in a reaction vessel under the following conditions: pressure between 0.1 and 50 bar (0.01 and 5 MPa), stirring of the reaction medium at 10 to 2000 rpm, temperature between -5 and 120 °C, stirring time between 0.01 and 24 h.

18. **THE PROCESS FOR THE COPRODUCTION OF OLEFINS AND C10 TO C13 ESTERS FROM METHYL FATTY ACID ESTERS** according to claim 17, **characterized in that** the metathesis reaction of olefins can be conducted in organic solvent and with inert gas.

19. **THE PROCESS FOR THE COPRODUCTION OF OLEFINS AND C10 TO C13 ESTERS FROM METHYL FATTY ACID ESTERS** according to claims 17 and 18, **characterized in that** the metathesis reaction of olefins can be preferably conducted with the organic solvent toluene.

20. **THE PROCESS FOR THE COPRODUCTION OF OLEFINS AND C10 TO C13 ESTERS FROM METHYL FATTY ACID ESTERS** according to claims 17, 18 and 19, **characterized in that** the inert gas is selected from nitrogen or argon with a purity greater than 99.9%.

21. **THE PROCESS FOR THE COPRODUCTION OF OLEFINS AND C10 TO C13 ESTERS FROM METHYL FATTY ACID ESTERS** according to claims 17 to 20, **characterized in that** the metathesis reaction of olefins is conducted in a reaction vessel under the following preferred conditions: pressure at 5 bar (0.5 MPa), stirring of the reaction medium at 500 rpm, temperature at 50 °C, stirring time 0.5 h.
